# EUROPÄISCHE PATENTANMELDUNG

(11) **EP 1 302 196 A1**
(43) Veröffentlichungstag der Anmeldung: **16.04.2003**
(21) Anmeldenummer: 02450235.3
(22) Anmeldetag: 16.10.2002
(51) Int. Cl.: A61K 7/16

(54) **Intelligenzfördernde Zahncreme**

(30) Priorität: 16.10.2001 AT 16412001
(71) Anmelder: Habinger, René, Ing., 1090 Wien (AT)
(72) Erfinder: Habinger, René, Ing., 1090 Wien (AT)

(57) **Zusammenfassung**

Die Erfindung bezieht sich auf die Anwendung eines pflanzlichen Phosphatidylserins in Kombination mit Mund- und Zahnpflegemittel, z.B. Zahnpasten, Zahngels, Zahnpulver, Mundwasser, Mundbad-Konzentrate, Mundspülmittel oder Kaugummi, usw., welche zur Verbesserung der Lernfähigkeit, Gedächnisleistung und Konzentrationsfähigkeit, zur Behandlung von Depressionen, zur Verringerung der Anfälligkeit gegenüber Stress, dem altersbedingten Abbau von Nervenzellen und zur Anwendung bei Alzheimer-Patienten dient. Durch diese Art der Anwendung wird Phosphatidylserin regelmäßig vom Körper aufgenommen. Weiters bezieht sich die Erfindung auf Mittel zur Mundpflege, die dadurch gekennzeichnet sind, dass sie Phosphatidylserin (kurz PS) als intelligenzfördemden Zusatzstoff, sowie andere zellstoffwechselanregende Zusatzstoffe, wie zum Beispiel Taurin, Ginseng, Guarana, Lecithin, Cholin, Coffein, Aminosäure, Selen, Glutamin(säure), Nicotinamid, Adenin und/oder Dinucleotid enthalten kann.

## Beschreibung

Es ist bekannt, dass einem Teil der Zahnpflegemittel Vitamine (bsw. A, B1, B5, B6, C, E,...) beigemengt werden. Diese Vitamine werden vom Körper aufgenommen und auch regelmässig benötigt.

Die Erfindung betrifft **Mund- und Zahnpflegemittel**, u.a. in Form aller für die Mundpflege geeigneten Präperate, wie Zahnpasten, Zahngels, Zahnpulver, Mundwasser, Mundbad-Konzentrate, Mundspülmittel oder Kaugummi, **mit** dem intelligenzfördernden Zusatzstoff Phosphatidylserin (kurz PS), sowie anderen **zellstoffwechselanregenden Zusatzstoffen**, wie zum Beispiel Taurin, Ginseng, Guarana, Lecithin, Cholin, Coffein, Aminosäure, Selen, Glutamin(säure), Nicotinamid, Adenin und/oder Dinucleotid.

Phosphatidylserin (PS) beispielsweise ist in den letzten 25 Jahren intensiv erforscht worden. Es ist wie Lecithin ein natürlich vorkommendes Phospholipid und wird in geringen Mengen auch vom Körper produziert. Phosphatidylserin ist im Gehirn ein wichtiger Regulator der normalen, gesunden Aktion der Neurotransmitter und des Informationsaustausches zwischen den Gehirnzellen (Neuronen).

Der vorliegenden Erfindung liegt die Aufgabe zugrunde, unter Beimengung von **Phosphatidylserin**, ein Zahnpflegemittel verfügbar zu machen, dass bei **regelmässiger Anwendung** zu einer signifikanten Verbesserung der Lernfähigkeit, Gedächtnisleistung, Konzentrationsfähigkeit und Abrufbarkeit von Gedächnisinhalten führen kann.

### Phosphatidylserin hat bei regelmässiger Anwendung die folgenden Wirkungen:

Unabhängig vom Alter und Gesundheitszustand kann jeder von Phosphatidylserin profitieren. Bei Schülern mit Lernschwächen konnten nach einer Therapie mit Phosphatidylserin deutliche **Verbesserungen der schulischen Leistungen** festgestellt werden. Wichtig ist zu erwähnen, dass sich durch Anwendung von Phosphatidylserin keinerlei Nebenwirkungen gezeigt haben.

Die regelmäßige Anwendung von Phosphatidylserin-Zahnpflegemittel verringert die Anfälligkeit gegenüber Stress, was sich besonders in Phasen erhöhter geistiger Beanspruchung am Arbeitsplatz positiv auswirkt. Phosphatidylserin moduliert die Freisetzung von Cortisol in Stress-Situationen.

Phosphatidylserin ist auch für **Kraftsportathleten** geradezu ideal, da es nicht den normalen Cortisonspiegel des Körpers beeinflusst, sondern ausschliesslich einen Cortisonanstieg bei körperlichen Streßsituationen verhindert.

Phosphatidylserin wird für die Behandlung von **Depressionen** und/oder verschlechterter mentaler Funktion eingesetzt. Hier verbessert Phosphatidylserin die depressiven Symptome und das Erinnerungsvermögen der Patienten. Im Unterschied zu den typischen Antidepressiva, führt Phosphatidylserin zu einer Verbesserung der Depressionen ohne jedoch negative Nebenwirkungen zu zeigen.

Phosphatidylserin wirkt als Therapeutikum gegen den **altersbdingten Abbau von Nervenzellen** (Dentriten) im Gehirn. Vor allem bei älteren Menschen mit altersassoziierten Gedächtnisstörungen konnten sehr gute Resultate zur Verbesserung von Lernfähigkeit, Gedächtnisleistung, Konzentrationsfähigkeit und Aufrufbarkeit von Gedächtnisinhalten erzielt werden.

Des weiteren weist Phosphatidylserin bei Alzheimer-Patienten eine positive Wirkung auf. In der Regel führt Phosphatidylserin bei **Alzheimer-Patienten** zu einer signifikanten Verbesserung von Angstsymptomen, Antrieb, Gedächtnisleistung und kognitiven Fähigkeiten.

Es hat sich gezeigt, dass sich durch Beimengung von Phosphatidylserin zu den üblichen Zahnund Mundhygieneprodukten der Mehrwert dieser Zahnpflegemittel, um die oben angeführten Eigenschaften erweitert werden kann. Das in den Zahnpflegemittel enthaltene Phosphatidylserin wird dabei sehr schnell absorbiert.

## Patentansprüche

1. Verwendung eines pflanzlichen Phosphatidylserins für die Herstellung eines Mund- oder Zahnpflegemittels zur Behandlung von Depressionen, zur Verbesserung der Lernfähigkeit, Gedächtnisleistung und Konzentrationsfähigkeit, zur Verringerung der Anfälligkeit gegenüber Stress, dem altersbedingten Abbau von Nervenzellen und zur Anwendung bei Alzheimer-Patienten.

2. Verwendung von Phosphatidylserin gemäß Anspruch 1, **dadurch gekennzeichnet, dass** dieses Phosphatidylserin einem Mund- oder Zahnpflegemittel, in Form aller für die Mundpflege geeigneten Präparate, wie Zahnpasten, Zahngels, Zahnpulver, Mundwasser, Mundbad-Konzentrate, Mundspülmittel oder Kaugummi beigemengt wird.

3. Mittel zur Mundpflege, wie Zahnpasten, Zahngels, Zahnpulver, Mundwasser, Mundbad-Konzentrate, Mundspülmittel oder Kaugummi, **dadurch gekennzeichnet, dass** es Phosphatidylserin (kurz PS) als intelligenzfördernden Zusatzstoff, sowie andere zellstoffwechselanregende Zusatzstoffe, wie zum Beispiel Taurin, Ginseng, Guarana, Lecithin, Cholin, Coffein, Aminosäure, Selen, Glutamin(säure), Nicotinamid, Adenin und/oder Dinucleotid enthalten kann.
